# EUROPÄISCHE PATENTSCHRIFT

(11) **EP 2 370 105 B1**
(45) Veröffentlichungstag und Bekanntmachung des Hinweises auf die Patenterteilung: **16.03.2016**
(21) Anmeldenummer: 09768501.0
(22) Anmeldetag: 07.12.2009
(51) Int. Cl.: A61K 47/48, A61K 49/00

(54) **Verbindungen bestehend aus einem Glykosaminoglykan und einer Stärke zum Transport eines kovalent gebundenen Arzneimittelwirkstoffes oder Fluoreszenzmarkers**
Compounds consisting of a glycosaminoglycan and a starch for transport of covalently bound medicaments or fluorescent markers
Composés consistant en un glycosaminoglycane et un amidon pour le transport d'un médicament ou d'un marqueur fluorescent lié de manière covalente

(30) Priorität: 06.12.2008 DE 102008060603; 08.07.2009 DE 102009032359; 31.03.2009 DE 102009015085
(43) Veröffentlichungstag der Anmeldung: 05.10.2011
(73) Patentinhaber: B. Braun Melsungen AG, 34212 Melsungen (DE)
(72) Erfinder: MEIER, Bernd, Horst, 64285 Darmstadt (DE); JANKOWIAK-MEIER, Iris, Theresia, 64285 Darmstadt (DE); MEIER, Clara, 64285 Darmstadt (DE); MEIER, Nele, 64285 Darmstadt (DE)
(74) Vertreter: von Kreisler Selting Werner - Partnerschaft von Patentanwälten und Rechtsanwälten mbB
(86) Internationale Anmeldenummer: PCT/EP2009/008718
(87) Internationale Veröffentlichungsnummer: WO 2010/063491

(56) Entgegenhaltungen:
- EP-A1- 1 152 013
- WO-A1-96/02260
- WO-A1-03/074087
- WO-A2-2009/135888
- DE-A1- 10 105 921
- DE-A1-102006 020 035
- GAMZE TORUM KOESE ET AL: "LOW-MOLECULAR-WEIGHT HEPARIN-CONJUGATED LIPOSOMES WITH IMPROVED STABILITY AND HEMOCOMPATIBILITY" DRUG DELIVERY, ACADEMIC PRESS, ORLANDO, FL, US, Bd. 5, Nr. 4, 1. Januar 1998 (1998-01-01), Seiten 257-264, XP009011091 ISSN: 1071-7544
- LEE ET AL: "Controlled dual release of basic fibroblast growth factor and indomethacin from heparin-conjugated polymeric micelle" INTERNATIONAL JOURNAL OF PHARMACEUTICS, ELSEVIER BV, NL LNKD- DOI:10.1016/J.IJPHARM.2007.06.025, Bd. 346, Nr. 1-2, 16. November 2007 (2007-11-16), Seiten 57-63, XP022348363 ISSN: 0378-5173

## Beschreibung

Die Erfindung betrifft an Transportvermittler gebundene Kolloide, die gegebenenfalls Arzneimittelstoffe oder Fluoreszenzmarker aufweisen, ein Verfahren zu deren Herstellung sowie eine pharmazeutische Zubereitung, die diese Verbindungen enthält.

Die kovalente Verbindung mit Kolloiden ermöglicht es Stoffe durch Phagozytose in Zellen des Immunsystems einzubringen, welche ohne diese Modifikation nicht bzw. nur verschwindend gering aufgenommen werden würden. In EP 1230935 A1 wird die chemische Bindung von arzneilich wirksamen Stoffen an ein Polysaccharid unter Ausbildung eines Linkers dargestellt. In ähnlicher Weise werden in WO2007122269 Arzneimittelwirkstoffe durch Ausbildung eines Linkers an Polysaccharid-Chitin bzw. Polysaccharid-Chitosan Verbindungen gebunden.

Die Aufnahme von Stoffen durch darauf spezialisierte Zellen des retikulohistiozytären Systems ist für eine Vielzahl von Kolloiden und Partikeln belegt. Die Einbringung von größeren Molekülen in nicht auf die Phagozytose spezialisierte Zellen des Körpers stellt jedoch ein Problem dar. Darüber hinaus werden durch Makrophagen phagozytierte Partikel und Kolloide nach Aufnahme in die Zelle sehr schnell in Lysosomen aufgenommen und dort durch eine Vielzahl lytischer Enzyme abgebaut. Das enzymatische Potential von Lysosomen ist hoch; eine Vielzahl von Arzneistoffen wird durch lysosomale Enzyme entsprechend schnell abgebaut. Von Chlamydia trachomatis ist bekannt, dass das Bakterium durch eukariotische Epithel-Zellen aufgenommen wird ohne in den Lysosomen enzymatisch abgebaut zu werden. Diese Aufnahme kann durch die Anwesenheit von Heparinen bzw Heparinsulfat signifikant vermindert werden. Stephens et al. (Infection and Immunity, March 2000, p. 1080-1085, Vol. 68, No. 3) zeigen, dass diesem Effekt eine Blockade der Heparin-Bindungsdomäne zugrunde liegt.

Im Gegenzug zeigen die Autoren, dass Polystyren-Mikrosphären nach Beschichtung mit Heparin durch Endozytose in eukaryotische Zellen aufgenommen werden. Heparin selbst bindet an eine Vielzahl von Enzymen. Patienten mit einer erhöhten Superoxiddismutaseaktivität im Blutserum weisen häufig eine mutierte Variante des Enzyms auf (CHU et al. Arteriosclerosis, Thrombosis, and Vascular Biology. 2006;26:1985).

Die mutierte Variante (R213G) besitzt auf der Position 213 statt der Aminosäure Arginin ein Glycin. Deshalb ist die Anbindung des Enzyms an Heparin nicht möglich. Die betroffenen Patienten weisen deshalb eine erhöhte Aktivität für das Enzym auf, weil das Enzym vermehrt im Serum und nicht in der Zelle vorliegt. Für Träger dieses Gendefektes bedeutet das ein 2,3fach erhöhtes Risiko für das Auftreten ischämischer Herzerkrankungen. EP 083 768 A1 beschreibt direkte Heparin-Protein Konjugate bei denen die terminale Aldose des Heparins an die N-terminale Aminogruppe eines Serpins gebunden ist, um den Effekt von Serpinen auf die Blutgerinnung und auf Atemnotsyndrome zu verstärken. Nach Kopplung an Heparine werden Proteine und Enzyme sehr schnell aus dem Serum eliminiert. Kleine Eiweiße unter 70 kDa verschwinden schon während der ersten Nierenpassage fast vollständig. Darüberhinaus sind für eine ganze Reihe von Proteinen Probleme bezüglich der Stabilität und Löslichkeit bekannt, die durch die kovalente Bindung an ein wasserlösliches Polysaccharid günstig beeinflusst werden können.

Heparin gehört zur Gruppe der Glucosaminoglykane, welche aus linearen Ketten sulfatierter Disaccharideinheiten bestehen. Jede Disaccharideinheit besteht aus je einer Hexuronsäure die wechselnd aus Glucuron- oder Iduronsäure und einer 2 Amino-2-desoxy-D-glucose bzw. N-sulfo-D-glucosamin zusammengesetzt ist. Wie Polysaccharide weisen Heparin und Glucosaminoglucane am terminalen Ende eine freie Aldehydgruppe auf. Heparin kommt intrazellulär fast ausschließlich in Mastzellen vor. Höher sulfatierte Heparine -Heparinsulfatewerden jedoch organübergreifend fast überall auf den Zellmembranen höherer Säuger gefunden.

Die gerinnungshemmende Wirkung des Heparins beruht in erster Linie auf seiner Affinität zu dem Serin-Protease-Inhibitor Antithrombin III. Die kleinste Heparineinheit, die diese Wirkung auf AT III besitzt, umfasst 5 Saccharideinheiten mit einer 3-O-Sulfatgruppe an der Glucosamingruppe. Dieses Pentasaccharid kann einen Heparin ATIII Komplex bilden, der den Gerinnungsfaktor Xa hemmt. Auch Thrombin kann durch die Bindung an spezifische Heparin-Strukturen inhibiert werden, die jedoch auf einem nur 5 Saccharideinheiten aufweisenden Pentasaccharid nicht vorhanden sind. Für die Hemmung von Thrombin sind Heparinverbindungen mit mindestens 18 Saccharideinheiten notwendig.

Molekülgröße und Sulfatierungsgrad haben nicht nur bei der selektiven Wirkung des Heparins auf Gerinnungsfaktoren entscheidende Bedeutung, sondern auch bei den Wechselwirkung mit einer Vielzahl körperlicher Cytokine und Wachstumsfaktoren. Eine Wechselwirkung von Heparin mit dem Fibroblast-Growth-Factor (FGF) setzt eine Mindestanzahl von 18 Glucosaminoglucaneinheiten mit einer spezifischen Sulfatierung des an C2 befindlichen Sauerstoffatoms voraus. Sulfatierte Heparine ab 8 Saccharideinheiten zeigen einen inhibitorischen Effekt auf die Angiogenese. Für diese sulfatierten Octasaccharide wird eine Behinderung der Gefäßeinsprossung in Tumorzellen diskutiert. Gleichzeitig scheinen diese Heparine keinen Effekt auf die Blutgerinnung zu haben. Molekülgröße und chemische Eigenschaften wie Anzahl und Lokalisation der Sulfat-, Carboxyl- und Aminogruppen haben entscheidenden Einfluss auf die Wirkung von Heparinen. Die Sulfatgruppen und in 2. Linie die Carboxylgruppen der Iduron- oder Glucurongruppen bewirken, dass Heparin eines der am stärksten elektronegativ geladenen Moleküle in Säugetieren ist. Die Molekülgröße sowie Menge und Position der Sulfat- und Carboxylgruppen erzeugen dabei ein spezifisches Ladungsmuster bzw. eine spezifische Ladungsverteilung der Heparinmoleküle. Die spezifische Ladungsverteilung spielt eine entscheidende Rolle bei der Affinität der Verbindungen sowohl an gerinnungsaktive Eiweiße und Proteasen, als auch an die Heparin-Bindungsdomänen von Endothelzellen. Die an Zellmembranen wirkende transportvermittelnde Funktion der Heparine ist noch stärker von der Ladungsstruktur des Glucosaminoglucans abhängig. Deshalb unterscheidet sich der kovalente Einbau von Heparinen in wasserlösliche Polysaccharide als Transportvermittler wesentlich vom Einbau der arzneilich wirksamen Stoffe. Das Heparin sollte möglichst dergestalt an das Kolloid gekoppelt werden, dass eine Bindungsstelle mit einer definierten Anzahl von Disaccharideinheiten und Ladungen stereoselektiv an die Heparin-Bindungsdomäne der Zellen passt. Das bedeutet, dass dieser Abschnitt des Heparins chemisch ungebunden und darüberhinaus vom restlichen Molekül sterisch unbehindert bleibt. Wie bei der spezifischen Wirkung des Heparins auf ATIII und Thrombin kommt auch hier der Ketteniänge und Anzahl der von dem Polysaccharid frei abstehenden sulfatierten Hexuronsäure und Aminodesoxyglucose eine grosse Bedeutung zu. Darüberhinaus gibt es Hinweise darauf, dass sich die für die Heparin-Bindungsdomänen relevanten Abschnitte des Heparinmoleküls eher in der Mitte des Heparinmoleküls befinden. Der zur beschriebenen Anlagerung an die Bindungsdomänen genutzte Teil des Moleküls ist aufgrund der Carboxyl- und Sulfatgruppen stark elektronegativ geladen. Die Blockade bzw. Störung dieses stereospezifisch relevanten Ladungsmusters durch die kovalente Verbindung mit dem Polysaccharid mittels eines Linkers sollte für diese Stellen möglichst unterbunden werden.

Heparin weist als streng lineares Glucosaminoglucan zur Bindung an andere Moleküle nutzbare funktionelle Gruppen auf. Im Bereich der Iduron- und Glucuronsäure liegen Carboxylgruppen an C6 vor. An C2 und C3 und C1 der ersten Saccharideinheit befinden sich Hydroxylgruppen. Jede 2. Saccharidgruppe trägt am C2-Atom eine Aminogruppe. Diese Aminogruppe und die Carboxylgruppe können sulfatiert sein. Schließlich trägt Heparin am terminalen Ende eine Aldehydgruppe.

Es ist bekannt, dass Punktmutationen einiger Proteine codierender Gene zu einem Austausch von Diamino-mono-carbonsäuren durch andere Aminosäuren führen. Bei einigen dieser Fälle (Superoxiddismutase) geht diese Veränderung der Aminosäuresequenz mit dem Verlust der Fähigkeit zur Bindung an Heparinsulfate einher, wodurch die Protein nicht mehr in die Zelle transportiert wird. Auch diese Befunde belegen, dass die Einbindung von Heparinen in Makromoleküle als Transportvermittler, also zum Zweck der Regulation des Durchtritts durch Zellmembranen seitens des Makromoleküls an regioselektive Bedingungen geknüpft ist. Der Verlust von nicht durch Peptidbindungen gebundenen Aminogruppen bedeutet hier den Verlust der Fähigkeit der Einbindung des Transportvermittlers Heparin. Mit der unspezifischen kovalenten Bindung an Heparin wird in der Medizintechnik häufig versucht die Bildung von Blutgerinnseln an Implantaten zu unterbinden.

Für die Einbringung von arzneilich wirksamen Stoffen in spezifische Organe und Zellsysteme des Körpers müssen die folgenden Bedingungen erfüllt sein:
1. Die Aufnahme des gebundenen Arzneistoffs erfolgt auch in Zellen, die nicht auf Phagozytose spezialisiert sind.
2. Nach dem Durchtritt durch die äußere Zellmembran soll der gebundene Arzneistoff nicht in Lysosomen aufgenommen, und nicht enzymatisch abgebaut werden.
3. Der Arzneistoff-Komplex, der aus Arzneistoff, der an einen Transportvermittler und/oder an ein Kolloid chemisch gebunden ist, besteht, sollte wasserlöslich sein hinreichend lange im Blut zirkulieren.
4. Der Arzneistoff-Komplex sollte keinen Einfluss auf die Blutgerinnung haben.
Überraschend wurde nunmehr gefunden, dass die Verknüpfung eines Transportvermittlers mit einem Kolloid (kolloidwirksame Verbindung) die zuvor genannten Probleme löst und insbesondere als geeignetes Transportsystem für kovalent daran geknüpfte Arzneistoffe und/oder Fluoreszenzmarker dient. Dies gilt insbesondere bei stereoselektiver Verknüpfung von Kolloid und Transportvermittler. Es wurde darüber hinaus überraschend gefunden, dass das Verknüpfungsprodukt an membranständige und intrazelluläre Bindungsdomänen binden kann, wenn die stereospezifischen Strukturen der Transportvermittler-Kolloid-Verbindung zur Anlagerung und Bindung an die zellulären Bindungsdomänen frei bleiben.

Gegenstand der vorliegenden Erfindung ist eine Verbindung der allgemeinen Formel (I)

(T-Z)ₙ-P (I),

wobei
- T ein Glykosaminoglykan als Transportvermittler,
- P eine kolloidwirksame Verbindung, ausgewählt aus Hydroxyalkylstärke und Carboxyalkylstärke, • Z ein erster Linker, durch den T und P kovalent verknüpft ist, und
- n eine ganze Zahl, die mindestens 1 beträgt,
und wobei der Transportvermittler T und/oder das Kolloid P m Gruppen -(L-A) trägt, wobei
- A ein Arzneimittelwirkstoff oder ein Fluoreszenzmarker,
- L ein zweiter Linker, durch den P mit A kovalent verknüpft ist, oder durch den T mit A kovalent verknüpft ist und
- m eine ganze Zahl, die mindestens 1 ist, ist.

Der Arzneimittelwirkstoff A wird ausgewählt aus der Gruppe bestehend aus Antibiotika, Chemotherapeutika, Cytostatika und cytotoxischen Substanzen. Bevorzugt weist der Transportvermittler T mindestens eine Bindungsstelle zur Anlagerung an zelluläre Bindungsdomänen auf.
Erfindungsgemäß unterscheiden sich die Tranportvermittler T von den Kolloiden P. Transportvermittler T im Rahmen der vorliegenden Erfindung begünstigen die Aufnahme in Zellen.

Als besonders geeignete Transportvermittler T haben sich die Glykosaminglykane oder Glykosaminglykanderivate herausgestellt. In einer bevorzugten Ausführungsform ist der Transportvermittler T daher ausgewählt aus der Gruppe bestehend aus Heparin und Heparinsulfat, insbesondere Heparin oder Heparinsulfat mit weniger als 6 Saccharideinheiten. Heparine mit weniger als 6 Saccharideinheiten als Transportvermittler haben den besonderen Vorteil, dass bei diesen Heparinen die möglicherweise auftretende Induktion von Autoantikörpern weitestgehend vermieden werden kann. Die kolloidwirksame Verbindung P (nachfolgend auch einfach "Kolloid P" genannt) ist bevorzugt ausgewählt aus der Gruppe bestehend aus Stärke und modifizierte Stärke. Stärken lassen sich beispielsweise durch Hydroxyalkylierung oder Veresterung modifizieren. Darüber hinaus können die Stärken auch aminiert werden, beispielsweise durch reduktive Aminierung.

Überaschend wurde gefunden, dass durch Aminierung des Kolloids P mittels reduktiver Aminierung, aminiert Kolloide, insbesondere modifizierte Stärken, wie etwa aminierte Hydroxyethylstärke oder aminierte Carboxymethylstärke erhalten werden können, die mit den Transportvermittlern mit hoher Stereoselektivität dergestalt eingebunden werden können, dass die entstehende Verbindung den von Körperzellen aufgenommenen Verbindungen aus Transportvermittler-Komplexen sehr ähnlich ist.

In einer bevorzugten Ausführungsform der vorliegenden Erfindung erfolgt die Verknüpfung von Transportvermittler und Kolloid stereoselektiv. Weiterhin ist es bevorzugt, wenn auch die Verknüpfung des Arnzeimittelwirkstoffs oder des Fluoreszenzmarkers stereoselektive mit dem Kolloid und/oder dem Transportvermittler erfolgt.

In einer bevorzugten Ausführungsform ist das Kolloid P ausgewählt aus der Gruppe bestehend aus Hydroxyalkylstärken, veresterte Stärken, Carboxyalkylstärken, Hydroxyalkyl-carboxyalkylstärke, aminierte Hydroxyalkylstärke, aminierte Hydroxyalkyl-carboxyalkylstärke und aminierte Carboxyalkylstärke.
Carboxyalkylstärken sind bevorzugt ausgewählt aus Carboxymethylstärke und Carboxyethylstärke.

Vorteilhaft können weitere spezielle Reste in die Kolloide mit eingeführt werden, die eine chemische Einbindung der arzneilich wirksamen Substanz oder des Fluoreszenzmarkers oder auch des Transportvermittlers erlauben, zum Beispiel Biotin, Aminosäuren oder Sulfidgruppen tragende Reste, wie Cystein.

Besonders bevorzugt handelt es sich im Rahmen der vorliegenden Erfindung bei dem Kolloid P um eine modifizierte Stärke ausgewählt aus der Gruppe bestehend aus Hydroxyethylstärke oder aminierter Hydroxyethylstärke, insbesondere eine Hydroxyethylstärke, die durch reduktive Aminierung aminiert wurde.

Die Hydroxyalkylgruppen in der Hydroxyethylstärke (HES) sind zur Behinderung des enzymatischen Abbaus der Stärke im Serum und zur Verbesserung der Wasserlöslichkeit in das Molekül eingeführt worden. Der Substitutionsgrad DS ist definiert das Verhältnis der Gesamtzahl der substituierten Monomereinheiten zur Gesamtzahl der Monomereinheiten. Im folgenden wird bei der Einführung von Substituenten ein Substitutionsgrad DS angegeben.

In einer weiteren Ausgestaltung der vorliegenden Erfindung weist die kolloidwirksame Verbindung ein mittleres Molekulargewicht von 20000 bis 800000 Dalton, vorzugsweise von 25000 bis 500000 Dalton, insbesondere von 30000 bis 200000 auf.

Der Substitutionsgrad DS der modifizierten Stärken, insbesondere der Hydroxyethylstärke, liegt bevorzugt zwischen 0,2 und 0,8, insbesondere zwischen 0,3 und 0,6.

Als Arzneistoffe A kommen alle Substanzen in Frage, die über einen Linker L an die oben genannten Kolloide und/oder Transportvermittler T eingebunden werden können.

Die erfindungsgemäßen Verbindungen können gegebenenfalls mit Arzneimittelwirkstoffen oder Fluoreszenzmarkern verknüpft sein. Bevorzugt ist der Arzneimittelwirkstoff ausgewählt aus der Gruppe bestehend aus Antibiotika, Chemotherapeutika, Cytostatika und cytotoxischen Substanzen.

Die Fluoreszenzmarker sind bevorzugt ausgewählt aus der Gruppe bestehend aus Fluoresceinisothiocyanat (FITC), Phycoerythrin, Rhodamid und 2-Aminopyridin.

Neben rein arzneilich wirkenden Stoffen können auch Fluoreszenzmarker z.B. Fluoresceinisothiocyanat in Verbindung mit dem Transportvermittler-Kolloid Komplex therapeutisch eingesetzt werden. Es ist bekannt, dass einige Tumore in größerer Zahl membrangebundene Bindungsdomänen exprimieren, beispielsweise um Anschluss an das Gefäßsystem (FGF-Rezeptoren) zu gewinnen. Die Markierung von für diese Bindungsdomänen spezifischen erfindungsgemäßen Transportvermittler-Kolloid-Komplexen mit Fluoreszenzmarkern wie Fluoreszeinisothiocyanat (A N. De Belder, K. Granath: Preparation and Properties of fluorescein-labelled dextrans, Carbohydrate Research, 30 (1973) 375-378) ermöglicht dem Chirurgen nach Injektion dieser Verbindung, Organanteile, die eine größere Zahl von Zellen mit diesen Bindungsdomänen aufweisen, optisch zu identifizieren (Near Infrared Fluorescence Imaging, NIRF).

In der Verbindung gemäß der Formel (I) (T-Z)ₙ-P ist der Transportvermittler T kovalent über eine erste Linkergruppe Z an das Kolloid P geknüpft. In einer bevorzugten Ausgestaltung der vorliegenden Erfindung ist der Linker Z eine funktionelle Gruppe, ausgewählt aus Carbonsäureester, Carbonsäureamide, Urethan, Ether und Amin oder umfasst zumindest eine solche funktionelle Gruppe. Insbesondere bevorzugt ist die kovalente chemische Bindung von T an P über den Linker Z reversibel, also ohne weiteres, beispielsweise enzymatisch, wieder spaltbar.

Der zweite Linker L, durch den das Kolloid P mit dem Arzneimittelwirkstoff oder dem Fluoreszenzmarker kovalent verknüpft ist oder durch den der Transportvermittler mit dem Arzneimittelwirkstoff oder dem Fluoreszenzmarker kovalent verknüpft ist, entspricht in seiner Funktion und Ausgestaltung ebenfalls dem ersten Linker Z. Für den Linker L ist es besonders vorteilhaft, wenn dieser ohne weiteres, beispielsweise enzymatisch, wieder spaltbar ist, wodurch es zur Freisetzung des Arzneimittelstoffs und/oder des Fluoreszenzmarkers kommt.

Die Bildung des Linkers Z oder L kann mit Hilfe der im Stand der Technik beschriebenen Methoden zur Bildung von Carbonsäureestern, Carbonsäureamide, Urethanen, Ethern und Aminen erfolgen.

In einer bevorzugten Ausführungsform ist die erfindungsgemäße Verbindung erhältlich durch Reaktion wenigstens einer freien
- Isocyanat-Gruppe (-NCO)
- Carboxyl-Gruppe (-COOH),
- Carbonsäurehalogenid-Gruppe (-CO-A, mit A = Cl, Br oder I),
- Alkylencarboxyl-Gruppe (-(CH₂)_{q}-COOH, mit q = 1-10 ),
- Ester-Gruppe (-COOR mit R = organischer Rest),
- Epoxy-Gruppe,
- oder nukleophilen Abgangsgruppe
des zugrundegelegten Kolloids P, mit einer freien

Hydroxyl-Gruppe (-OH)

des zugrundegelegten Transportvermittlers T, unter Bildung des Linkers Z, wobei das Kolloid P und/oder der Transportvermittler T mit m Resten -(L-A) verknüpft ist.

In einer weiteren Ausgestaltung der vorliegenden Erfindung ist die erfindungsgemäße Verbindung erhältlich durch Reaktion wenigstens einer freien
- Hydroxyl-Gruppe (-OH)
des zugrundeliegenden Kolloids P, mit einer freien
- Isocyanat-Gruppe (-NCO)
- Carboxyl-Gruppe (-COOH),
- Carbonsäurehalogenid-Gruppe (-CO-A, mit A = Cl, Br oder I),
- Alkylencarboxyl-Gruppe (-(CH₂)_{q}-COOH, mit q = 1-10 ),
- Ester-Gruppe (-COOR mit R = organischer Rest),
- Epoxy-Gruppe,
- oder nukleophilen Abgangsgruppe
des zugrundeliegenden Transportvermittlers T, unter Bildung des Linkers Z, wobei das Kolloid P und/oder der Transportvermittler T mit m Resten -(L-A) verknüpft ist.

In einer weiteren Ausgestaltung der vorliegenden Erfindung ist die erfindungsgemäße Verbindung erhältlich durch Reaktion wenigstens einer freien
- Amino-Gruppe (-NH₂)
des zugrundeliegenden Kolloids P, mit einer freien
- Isocyanat-Gruppe (-NCO)
- Carboxyl-Gruppe (-COOH),
- Carbonsäurehalogenid-Gruppe (-CO-A, mit A = Cl, Br oder I),
- Alkylencarboxyl-Gruppe (-(CH₂)_{q}-COOH, mit q = 1-10),
- Ester-Gruppe (-COOR mit R = organischer Rest),
- Epoxy-Gruppe,
- oder nukleophilen Abgangsgruppe
des zugrundeliegenden Transportvermittlers T, unter Bildung des Linkers Z, wobei das Kolloid P und/oder der Transportvermittler T mit m Resten -(L-A) verknüpft ist.

Weiterhin ist in einer bevorzugten Ausgestaltung die erfindungsgemäße Verbindung erhältlich durch Reaktion wenigstens einer freien
- Isocyanat-Gruppe (-NCO)
- Carboxyl-Gruppe (-COOH),
- Carbonsäurehalogenid-Gruppe (-CO-A, mit A = Cl, Br oder I),
- Alkylencarboxyl-Gruppe (-(CH₂)_{q}-COOH, mit q = 1-10 ),
- Ester-Gruppe (-COOR mit R = organischer Rest),
- Epoxy-Gruppe,
- oder nukleophilen Abgangsgruppe
des zugrundeliegenden Kolloids P, mit einer freien
- Amino-Gruppe (-NH₂)
des Transportvermittlers T, unter Bildung des Linkers Z, wobei das Kolloid P und/oder der Transportvermittler T mit m Resten -(L-A) verknüpft ist.

Besonders bevorzugt ist die erfindungsgemäße Verbindung erhältlich durch Reaktion wenigstens einer freien
- Hydroxyl-Gruppe (-OH) oder
- Amino-Gruppe (-NH₂)
des zugrundeliegenden Kolloids P, mit einer freien
- Isocyanat-Gruppe (-NCO)
- Carboxyl-Gruppe (-COOH),
- Carbonsäurehalogenid-Gruppe (-CO-A, mit A = Cl, Br oder I),
- Alkylencarboxyl-Gruppe (-(CH₂)_{q}-COOH, mit q = 1-10 ),
- Ester-Gruppe (-COOR mit R = organischer Rest),
- Epoxy-Gruppe,
- oder nukleophilen Abgangsgruppe
des zugrundeliegenden Transportvermittlers T, unter Bildung des Linkers Z, wobei das Kolloid P und/oder der Transportvermittler T mit m Resten -(L-A) verknüpft ist.
Im Rahmen der vorliegenden Erfindung sind nukleophile Abgangsgruppen bevorzugt ausgewählt aus der Gruppe der Halogenide und Tosylate.
Weiterhin können die erfindungsgemäßen Verbindungen erhältlich sein durch Reaktion eines Diamins der allgemeinen Formel II

R¹(-NH₂)₂ (II),

wobei R¹ ausgewählt ist aus
- einer Einfachbindung
- linearen oder verzweigten, gesättigten oder ungesättigten, aliphatischen oder alicyclischen Kohlenwasserstoffresten mit 1 bis 22 Kohlenstoffatomen;
- Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
- Heteroaryl-, Heteroaryl-C₁-C₄-Alkyl- und Heteroaryl-C₂-C₆-Alkenyl-gruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxy-gruppen,
mit einer freien funktionellen Gruppe des zugrundeliegenden Transportvermittlers T und wenigstens einer freien funktionellen Gruppe des zugrundeliegenden Kolloids P, die jeweils unabhängig voneinander ausgewählt sind aus
- Isocyanat-Gruppe (-NCO)
- Carboxyl-Gruppe (-COOH),
- Carbonsäurehalogenid-Gruppe (-CO-A, mit A = Cl, Br oder I),
- Alkylencarboxyl-Gruppe (-(CH₂)_{q}-COOH, mit q = 1-10 ),
- Ester-Gruppe (-COOR mit R = organischer Rest),
- Epoxy-Gruppe,
- oder nukleophilen Abgangsgruppe
unter Bildung des Linkers Z, wobei das Kolloid P und/oder der Transportvermittler T mit m Resten -(L-A) verknüpft ist.

Geeignete Diamine sind beispielsweise 1,2-Diaminoethan, 1,2- oder 1,3-Diaminopropan, 1,2-, 1,3- oder 1,4-Diaminobutan, 1,5-Diaminopentan, 2,2-Dimethyl-1,3-diaminopropan, Hexamethylendiamin, 1,7-Diamino-heptan, 1,8-Diaminooctan, Trimethyl-1,6-diaminohexan, 1,9-Diaminononan, 1,10-Diaminodecan, 1,12-Diaminododecan, 1,2-Diaminocyclohexan, 1,4-Diaminocyclohexan, 1,3-Cyclohexanbis(methylamin), 1,2-Phenylendiamin, 1,3-Phenylendiamin, 1,4-Phenylendiamin, 4,4'-Ethylendianilin, 4,4'-Methylendianilin, 4,4'-Diaminostilben, 4,4'-Thiodianilin, 4-Aminophenyldisulfid, 2,6-Diamino-pyridin, 2,3-Diaminopyridin, 3,4-Diaminopyridin, 2,4-Diaminopyrimidin, 4,5-Diaminopyrimidin, 4,6-Diaminopyrimidin.

Darüber hinaus lassen sich in einer weiteren Ausgestaltung der vorliegenden Erfindung die erfindungsgemäßen Verbindungen erhalten durch Reaktion eines Diols der allgemeinen Formel III

R²(-OH)₂ (III),

wobei R² ausgewählt ist aus
- linearen oder verzweigten, gesättigten oder ungesättigten, aliphatischen oder alicyclischen Kohlenwasserstoffresten mit 2 bis 22 Kohlenstoffatomen;
- Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
- Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit einer freien funktionellen Gruppe des zugrundeliegenden Transportvermittlers T und wenigstens einer freien funktionellen Gruppe des zugrundeliegenden Kolloids P, die jeweils unabhängig voneinander ausgewählt sind aus
- Isocyanat-Gruppe (-NCO)
- Carboxyl-Gruppe (-COOH),
- Carbonsäurehalogenid-Gruppe (-CO-A, mit A = Cl, Br oder I),
- Alkylencarboxyl-Gruppe (-(CH₂)_{q}-COOH, mit q = 1-10 ),
- Ester-Gruppe (-COOR mit R = organischer Rest),
- Epoxy-Gruppe,
- oder nukleophilen Abgangsgruppe
unter Bildung des Linkers Z, wobei das Kolloid P und/oder der Transportvermittler T mit m Resten -(L-A) verknüpft ist.

Geeignete Diole sind beispielsweise Ethylenglycol, Propylenglycol, Butylenglycol, und Neopentylglycol, Pentandiol-1,5,3-Methylpentandiol-1,5, Bisphenol A, 1,2-oder 1,4-Cyclohexandiol, Caprolactondiol (Reaktionsprodukt aus Caprolacton und Ethylenglycol), hydroxyalkylierte Bisphenole, Trimethylolpropan, Trimethylolethan, Pentaerythritol, Hexandiol-1,6, Heptandiol-1,7, Octandiol-1,8, Butandiol-1,4, 2-Methyloctandiol-1,8, Nonandiol-1,9, Decandiol-1,10, Cyclohexandimethylol, Di-, Tri- und Tetraethylenglykol, Di-, Tri- und Tetrapropylenglykol, Polyethylen- und Polypropylenglykole mit einem mittleren Molekulargewicht von 150 bis 15.000.

In einer weiteren Ausgestaltung der vorliegenden Erfindung sind die erfindungsgemäßen Verbindungen erhältlich durch Reaktion einer Dicarbonsäure der allgemeinen Formel IV

R³(-COOH)₂ (IV)

wobei R³ ausgewählt ist aus
- einer Einfachbindung
- linearen oder verzweigten, gesättigten oder ungesättigten, aliphatischen oder alicyclischen Kohlenwasserstoffresten mit 1 bis 22 Kohlenstoffatomen;
- Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
- Heteroaryl-, Heteroaryl-C₁-C₄-Alkyl- und Heteroaryl-C₂-C₆-Alkenyl-gruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxy-gruppen,
mit jeweils einer freien funktionellen Gruppe des zugrundeliegenden Transportvermittlers T und wenigstens einer freien funktionellen Gruppe des zugrundeliegenden Kolloids P, die jeweils unabhängig voneinander ausgewählt sind aus
- Amino-Gruppe (-NH₂),
- oder Hydroxyl-Gruppe (-OH),
unter Bildung des Linkers Z, wobei das Kolloid P und/oder der Transportvermittler T mit m Resten -(L-A) verknüpft ist.

Geeignete Dicarbonsäuren sind beispielsweise Oxalsäure, Malonsäure, Bernsteinsäure, Glutarsäure, Adipinsäure, Pimelinsäure, Azelainsäure, Sebacinsäure, Maleinsäure, Fumarsäure, Sorbinsäure, Phthalsäure, Terephthalsäure, Isophthalsäure oder Agaricinsäure.

Insbesondere können die erfindungsgemäßen Verbindungen auch erhältlich sein durch Reaktion eines Dicarbonsäurehalogenids der allgemeinen Formel V

R⁴(-CO-A)₂, (V),

wobei A = Cl, Br oder I und R¹ ausgewählt ist aus
- einer Einfachbindung;
- linearen oder verzweigten, gesättigten oder ungesättigten, aliphatischen oder alicyclischen Kohlenwasserstoffresten mit 1 bis 22 Kohlenstoffatomen;
- Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
- Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit einer freien funktionellen Gruppe des zugrundeliegenden Transportvermittlers T und wenigstens einer freien funktionellen Gruppe des zugrundeliegenden Kolloids P, die jeweils unabhängig voneinander ausgewählt sind aus
- Amino-Gruppe (-NH₂),
- oder Hydroxyl-Gruppe (-OH),
unter Bildung des Linkers Z, wobei das Kolloid P und/oder der Transportvermittler T mit m Resten -(L-A) verknüpft ist.

Darüber hinaus sind in einer weiteren bevorzugten Ausgestaltung die erfindungsgemäßen Verbindungen erhältlich durch Reaktion eines Diesters der allgemeinen Formel VI

R⁵(-COOR')₂ (VI)

wobei R' eine C₁₋₁₀-Alkylgruppe ist und R⁵ ausgewählt ist aus
• einer Einfachbindung;
• linearen oder verzweigten, gesättigten oder ungesättigten, aliphatischen oder alicyclischen Kohlenwasserstoffresten mit 1 bis 22 Kohlenstoffatomen;
• Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
• Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alky)- und/oder C₂-C₆-Alkoxygruppen,
mit jeweils einer freien funktionellen Gruppe des zugrundeliegenden Transportvermittlers T und wenigstens einer freien funktionellen Gruppe des zugrundeliegenden Kolloids P, die jeweils unabhängig voneinander ausgewählt sind aus
- Amino-Gruppe (-NH₂),
- oder Hydroxyl-Gruppe (-OH),
unter Bildung des Linkers Z, wobei das Kolloid P und/oder der Transportvermittler T mit m Resten -(L-A) verknüpft ist.

Weiter bevorzugt sind die erfindungsgemäßen Verbindungen erhältlich durch Reaktion eines Diisocyanats der allgemeinen Formel VII

R⁶(-NCO)₂ (VII),

wobei R⁶ ausgewählt ist aus
- linearen oder verzweigten, gesättigten oder ungesättigten, aliphatischen oder alicyclischen Kohlenwasserstoffresten mit 1 bis 22 Kohlenstoffatomen;
- Aryl-, Aryl-C₁-C₄-Alkyl- und Aryl-C₂-C₆-Alkenylgruppen mit 5 bis 12 Kohlenstoffatomen im Arylrest, die gegebenenfalls substituiert sein können mit C₁-C₆-Alkyl- und/oder C₂-C₆-Alkoxygruppen; oder
- Heteroaryl-, Heteroaryl-C₁-C₄-Alkyl- und Heteroaryl-C₂-C₆-Alkenylgruppen mit 3 bis 8 Kohlenstoffatomen im Heteroarylrest und einem oder zwei Heteroatom(en) ausgewählt aus N, O und S, die substituiert sein können mit C₁-C₆-Alkyl- und/oder C₂-C₆-Alkoxygruppen,
mit jeweils einer freien funktionellen Gruppe des zugrundeliegenden Transportvermittlers T und wenigstens einer freien funktionellen Gruppe des zugrundeliegenden Kolloids P, die jeweils unabhängig voneinander ausgewählt sind aus
- Amino-Gruppe (-NH₂),
- oder Hydroxyl-Gruppe (-OH),
unter Bildung des Linkers Z, wobei das Kolloid P und/oder der Transportvermittler T mit m Resten -(L-A) verknüpft ist.

Geeignete Diisocyanate sind beispielsweise Toluylendiisocyanat, Bitoluylendiisocyanat, Dianisidindiisocyanat, Tetramethylendiisocyanat, Hexamethylendiisocyanat, m-Phenylendiisocyanat, m-Xylylendiisocyanat, C₁-C₆ Alkylbenzoldiisocyanat, 1-Chlorobenzol-2,4-diisocyanat, Cyclohexylmethandiisocyanat, 3,3'-Dimethoxydiphenylmethan-4,4'-diisocyanat, 1Nitrobenzol-2,4diisocyanat, 1-Alkoxybenzol-2,4-diisocyanat, Ethylendiisocyanat, Propylendiisocyanat, Cyclohexylen-1,2-diisocyanat, 3,3'-Dichloro-4,4'-biphenylendiisocyanat, Diphenylendiisocyanat, 2-Chlorotrimethylendiisocyanat, Butylen-1,2-diisocyanat, Ethylidendiisocyanat, Diphenylmethan-4,4'diisocyanat, Diphenylethandiisocyanat, 1,5-Naphthalindiisocyanat, Cyclohexandiisocyanat und Isophorondiisocyanat.

Insbesondere bevorzugt ist die erfindungsgemäße Verbindung erhältlich durch Reaktion eines Diepoxids mit jeweils einer freien funktionellen Gruppe des zugrundeliegenden Transportvermittlers T und wenigstens einer freien funktionellen Gruppe des zugrundeliegenden Kolloids P, die jeweils unabhängig voneinander ausgewählt sind aus
- Amino-Gruppe (-NH₂),
- oder Hydroxyl-Gruppe (-OH),
unter Bildung des Linkers Z, wobei das Kolloid P und/oder der Transportvermittler T mit m Resten -(L-A) verknüpft ist.

Als geeignete Diepoxide haben sich insbesondere 1,2,3,4-Diepoxybutan oder 1,2,7,8-Diepoxyoctan erwiesen.

Als besonders vorteilhaft haben sich Verbindungen herausgestellt, bei denen die Verknüpfung von Transportvermittler T und Kolloid P über eine reduktive Aminierung erfolgt. Besonders bevorzugt sind die erfindungsgemäßen Verbindungen somit erhältlich durch reduktive Aminierung eines freie Aminogruppen (-NH₂) aufweisenden Kolloids P mit einem Transportvermittler T, der mindestens eine Aldehyd- oder Ketogruppe aufweist und wobei das Kolloid P und/oder der Transportvermittler T mit m Resten -(L-A) verknüpft ist.

Das Aminogruppen aufweisende Kolloid P ist hierbei bevorzugt ausgewählt aus der Gruppe bestehend aus aminierter Stärke, aminierter Hydroxyalkylstärke, aminierter Hydroxyalkylcarboxyalkylstärke und aminierter Carboxyalkylstärke. Insbesondere bevorzugt ist aminierte Hydroxyalkylstärke, die beispielsweise selbst durch reduktive Aminierung erhalten werden kann.

Die durch reduktive Aminierung verknüpften Kolloide P mit Transporvermittlern T weisen besonders bevorzugt Transportvermittler, ausgewählt aus der Gruppe von Heparin oder Heparinderivaten auf.

In einer besonders bevorzugten Ausgestaltung der vorliegenden Erfindung ist der Transportvermittler T Heparin und das Kolloid P eine Hydroxyethylstärke und der ersten Linker Z eine -NH-Gruppe.

Wie bereits oben erörtert, ist der zweite Linker L bevorzugt eine funktionelle Gruppe ausgewählt aus Carbonsäureester, Carbonsäureamid, Urethan, Ether und Amin oder umfasst zumindest eine solche funktionelle Gruppe.

Des weiteren können auch mehrere Kolloide P über die Linker-Moleküle L und/oder Z zu größeren Clustern verknüpft werden. Diese Reaktion kann in Konkurrenz zur Anbindung A und/oder T an das Kolloid P treten. Erfindungsgemäß kann das Verhältnis dieser konkurrierenden Reaktionen durch geeignete Modifikation des verwendeten Verfahrens beeinflusst werden. Dies kann am einfachsten durch Veränderung des Verhältnisses der eingesetzten Reagenzien und Substrate sowie durch Modifikation des Molgewichtes des Kolloids erfolgen. Des weiteren beeinflussen auch Reaktionsbedingungen wie Temperatur, Druck und Katalysatoren das Verhältnis der beiden Reaktionen.

Das Kolloid P kann ein oder mehrere über den ersten Linker Z verknüpfte Transportvermittler T aufweisen. Die Anzahl der mit dem Kolloid P verknüpften Transportvermittler T wird durch den Parameter n definiert. In einer bevorzugten Ausgestaltung der vorliegenden Erfindung ist n eine ganze Zahl zwischen 1 und 10000, bevorzugt 2 und 1000, weiter bevorzugt zwischen 5 und 500, insbesondere zwischen 10 und 100.

In einer weiteren bevorzugten Ausgestaltung der vorliegenden Erfindung sind der Transportvermittler T und/oder das Kolloid P über den zweiten Linker L kovalent mit A, d.h. dem Arzneimittelwirkstoff oder dem Fluoreszenzmarker verknüpft. In einer bevorzugten Ausführungsform der vorliegenden Erfindung ist daher der Paramter m eine ganze Zahl mit mindestens 1, vorzugsweise ist m eine ganze Zahl zwischen 1 und 10.000, weiter bevorzugt zwischen 2 und 1000, noch weitere bevorzugt zwischen 5 und 500, insbesondere zwischen 10 und 100.

Ein weiterer Gegenstand der vorliegenden Erfindung ist eine pharmazeutische Zubereitung umfassend die erfindungsgemäße Verbindung.

Die erfindungsgemäße pharmazeutische Zubereitung ist bevorzugt eine wässrige Zubereitung und weiter bevorzugt injizierbar. Vorzugsweise liegt die erfindungsgemäße Verbindung in einer Konzentration von 0,0001 bis 50 Gew.-%, insbesondere 0,01 bis 10 Gew.-%, beispielsweise 0,1 bis 5,5 Gew.-%, jeweils bezogen auf die Gesamtzusammensetzung, vor.

Ein weiterer Gegenstand der vorliegenden Erfindung ist ein Verfahren zur Herstellung der erfindungsgemäßen Verbindung durch Verknüpfen eines Transportvermittlers T mit einer kolloidwirksamen Verbindung P unter Ausbildung eines Linkers Z, durch den T und P kovalent miteinander verknüpft sind und wobei das Kolloid P und/oder der Transportvermittler T mit m Resten (L-A) verknüpft ist. Die Bedeutung von T, P, Z, L und A entspricht den vorgenannten Definitionen.

In einer bevorzugten Ausführungsform des erfindungsgemäßen Verfahrens erfolgt die Verknüpfung von Transportvermittler T mit dem Kolloid P durch reduktive Aminierung.

Es ist hierbei bevorzugt, dass in einem ersten Schritt das Kolloid P ausgewählt ist aus der Gruppe bestehend aus aminierter Stärke, aminierter Hydroxyalkylstärke, aminierter Hyxdroxycarboxylalkylstärke und aminierter Carboxyalkylstärke mit einem Transportvermittler T, ausgewählt aus der Gruppe der Heparine oder Heparinderivate, in Gegenwart eines Reduktionsmittels zur Reaktion gebracht wird.

Bevorzugt ist das Reduktionsmittel ausgewählt aus der Gruppe bestehend aus LiAlH₄, LiBH₄, NaBH₄ und NaBCNH₃.

In einer weiteren bevorzugten Ausgestaltung des erfindungsgemäßen Herstellverfahrens erfolgt zunächst in einem vorgelagerten Schritt eine reduktive Aminierung einer modifizierten Stärke, bevorzugt einer Hydroxyalkylstärke oder einer Carboxymethylstärke. Die reduktive Aminierung erfolgt vorteilhaft mit Ammoniak oder Ammoniumhydroxid in Anwesenheit eines Katalysators. Diese Reaktion erfolgt bevorzugt in einer Wasserstoffatmosphäre unter erhöhtem Druck, beispielsweise 10 bis 300 bar, vorzugsweise 20 bis 100 bar und Temperaturen im Bereich von 50 bis 300 °C, vorzugsweise 80 bis 200 °C. Als Katalysatoren werden Raney, Nickel- bzw. Kobaltnickelkatalysatoren verwendet. Die so erhältliche aminierte modifizierte Stärke kann anschließend mit einem Transportvermittler, beispielsweise einem Glucosaminoglucan in einer weiteren reduktiven Aminierungsreaktion verknüpft werden.

In einer weiteren Ausgestaltung des erfindungsgemäßen Verfahrens der vorliegenden Erfindung wird der Transportvermittler T, bevorzugt das Glucosaminoglucan, insbesondere das Heparin oder das Heparinderivat in einem ersten Schritt auf einem elektrisch geladenen Träger aufgebracht. Anschließend wird die weitere Kopplungsreaktion mit dem Kolloid P durchgeführt, wobei der der Transportvermittler T auf dem geladenen Träger belassen wird. Das Aufbringen des Transportvermittlers auf den geladenen Träger hat sich als vorteilhaft herausgestellt, da sich die ionisch geladenen Bereiche des Transportvermittlers T, insbesondere des Heparins oder eines Heparinderivats in Richtung des Ladungsträgers orientieren und daher diese Bereiche für die Kopplungsreaktion mit dem Kolloid P nur schwer zugänglich sind. Es lassen mit diesem Verfahren somit selektiv spezifische Bindungsdomänen des Transportvermittlers erhalten.

In einer besonderen Ausführungsform der vorliegenden Efindung wird als Transportvermittler Heparin oder ein Heparinderivat und als Kolloid Hydroxyethylstärke oder Carboxymethylstärke eingesetzt.

Bevorzugt wird der zur Assoziation an die Heparin-Bindungsdomäne vorgesehene Teil des Heparinmoleküls an einen bevorzugt nanostrukturierten, weiter bevorzugt positv geladenen Körper angelagert und von der kovalenten Einbindung in das Hydroxyethylstärke und/oder Carboxymethylstärke Molekül freigehalten. Beispielsweise kann hierzu eine Kupferplatte mit einem Isolator beschichtet und mit einem Laser an ausgewählten Stellen von der Isolatorschicht befreit werden. Besonders vorteilhaft können die Ladungsstrukturen der Heparin-Bindungsdomänen durch Rasterelektronenmikroskopie erfasst und entsprechende Ladungsmuster zur Immobilisation der Heparinmoleküle in die Isolatorschicht gebrannt werden. Auch kann die Rasterelektronenmikroskopie zur Einbringung geeigneter Ladungsmuster in die Isolatorschicht des Anlagerungskörpers genutzt werden. Dem Fachmann sind entsprechende Anwendungen der Lasertechnik bekannt. Das Aufbringen des Transprotvermittlers auf eine geladene Oberfläche kann auch direkt durch Aufbringen auf andere positiv geladenen Moleküle erfolgen. Besonders geeignet sind hierfür stark positiv geladene Polymere, die unter den Reaktionsbedingungen der erfindungsgemäßen Synthesen als Film und Polykation vorliegen. Bei Linkern, die unter alkalischen Reaktionsbedingungen reagieren liegt beispielsweise Chitosan als Polykation vor, an das Heparin sich gut anlagert. Hier ist zu beachten, dass bei der kovalenten Bindung des Heparins an das Kolloid P, insbesondere die Hydroxyethylstärke, keine Verbindung zwischen einer funktionellen Gruppe des Chitosans mit dem Polysaccharid erfolgen soll, sondern nur zwischen der Hydroxyethylstärke und dem Heparin.

Die Erfindung wird durch die nachfolgenden Beispiele näher erläutert.

### Beispiele

### Beispiel 1a (Verknüpfung von mit Fluorescein-Isothiocyanat gekoppelter Hydroxyethylstärke (FITC-HES) mit Heparin)

200 mg Heparin¹⁾ werden in 10 ml PBS (Phosphate buffered saline; phosphatgepufferte Salzlösung); pH 7,5 gelöst und auf eine mit einem Bandgenerator positiv aufgeladene Platte pipettiert. Die Platte wird für 1 Woche geladen gehalten bis die Lösung als Film getrocknet ist. Auf den Film werden 0,2 ml 1,2,7,8-Diepoxyoctan (ex ALFA-AESAR GmbH & Co.KG, Deutschland) pipettiert und durch Rotation verteilt. 80 mg Hydroxyethylstärke (HES) [mittleres Molekulargewicht: 50 kDa; DS = 0,4] werden nach dem von DeBelder und Granath beschriebenen Verfahren²⁾ mit Fluorescein-Isothiocyanatresten kovalent verbunden (FITC-HES). Die FITC-HES wird in 10 ml einer Mischung aus 3 ml 1N NaOH und 7 ml Aceton gelöst und unter Schütteln auf die geladene Platte aufgetropft. Das Gemisch wird auf einen pH-Wert von 10 eingestellt und in einem abgedunkelten Raum alle 30 Minuten geschüttelt. Nach 12 Stunden wird die Lösung entnommen gegen destilliertes Wasser dialysiert und anschließend gefriergetrocknet. Das Reagenz wird in 10 ml PBS; pH=7,5 aufgenommen. In einer Elektrophorese wandert das Verknüpfungsprodukt signifikant schneller als eine nicht mit Heparin geknüpfte FITC-HES.

### Beispiel 1 b) (Wirksamkeit des FITC-HES-Heparin Verbindung aus Beispiel 1a))

100 mg Trockensubstanz der in Bespiel 1a) synthetisierten FITC-HES-Heparin Verbindung werden in 5 ml einer wässrigen 0,9 %-igen NaCl Lösung gelöst und einer Wistar Ratte i.p. injiziert. Nach 6 Stunde wurde das Tier unter Narkose getötet und die Organe entnommen.

Aus der Milz wurde eine 0,6 X 0,8 cm großes Gewebestück entnommen und über Nacht in Formalin eingelegt. Nach einer aufsteigenden Ethanolreihe und Methylbenzoat-Reihe wurden 6-8 µm dicke Schnitte angefertigt. Die Präparate wurden mit einem Fluoreszenzmikroskop mit einer Wellenlänge von 450-490 nm mikroskopiert.

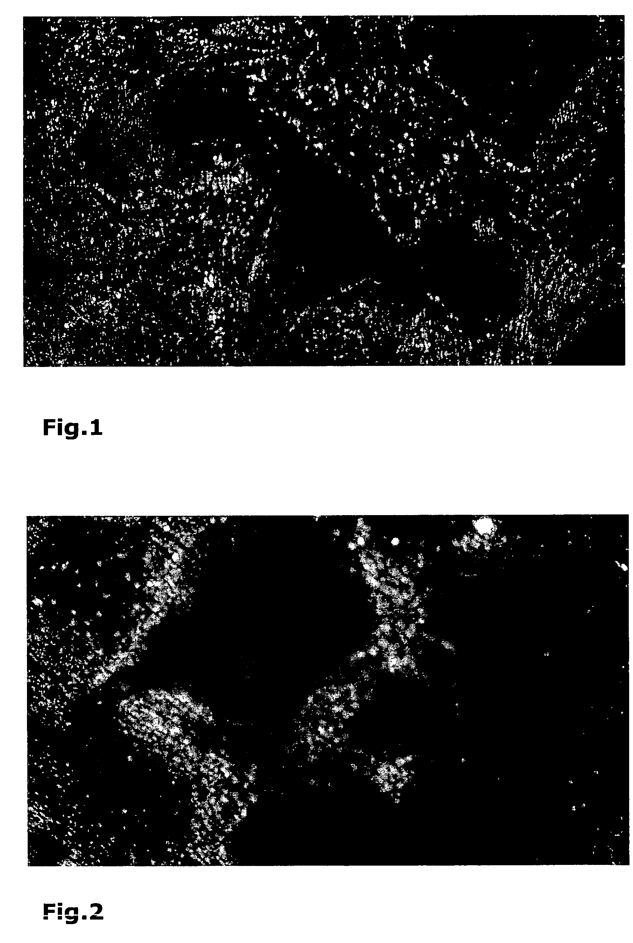

Figuren 1 und 2 zeigen in 20-facher Vergrößerung eine deutliche Fluoreszenz der Zellen. Die hellen Bereiche in den Fotos belegen die Aufnahme des fluoreszenzmarkierten HES-Heparin-Komplexes in den Gewebezellen der Milz.

### Beispiel 2 (Verknüpfung von Carboxymethyl-Hydroxyethylstärke mit Heparin)

200 mg Heparin¹⁾ werden in destilliertem Wasser gelöst und wie in Beispiel 1 a) behandelt. 10 ml einer 6%-igen Carboxmethyl-Hydroxyethylstärke [DS für Carboxymethylgruppen: 0,06 und DS für die Hydroxyethylgruppen: 0,34] werden in 10 ml einer 0,1N HCl Acetonlösung (3 ml 0,1 N HCl und 7 ml Aceton) gelöst und zusammen mit 0,2 ml 1,2,7,8-Diepoxyoctan zugegeben und geschüttelt.

Das Gemisch wird durch Zugaben der HCl/Aceton Lösung auf einen pH zwischen 3 und 4 eingestellt und alle 30 Minuten geschüttelt. Nach 12 Stunden wird die Lösung entnommen gegen destilliertes Wasser dialysiert und anschließend gefriergetrocknet.

### Beispiel 3 (Verknüpfung einer aminierten HES mit Heparin)

200 g einer Hydroxyethylstärke (HES) [mittleres Molekulargewicht Mw = 50.000; DS = 0,3] wird zusammen mit einer 27%igen Ammoniumhydroxidlösung und 300 g eines Nickel/Kupfer/Chrom Katalysators mit einem Nickelanteil von 75%, einem Kupferanteil von 23% und einem Chromanteil von 2% in einen Autoclaven eingebracht. Unter Zugabe von Wasserstoff wird über einen Zeitraum von 12 Stunden mit Überdruck beaufschlagt. Die Temperatur wird bis auf 220 °C eingestellt. Anschließend wird das Gemisch entnommen, dialysiert und gefriergetrocknet. 200 mg Heparin¹⁾ werden in 5ml PBS; pH= 7,5 gelöst und auf ein mit einem Bandgenerator positiv geladenes Petriglas pipettiert. 200 mg der reduktiv aminierten Hydroxyethylstärke werden in 10 ml destilliertes Wasser gelöst und vorsichtig zugegeben. Danach werden 0,025 mg Natriumcyanoborhydrid NaBH₃CN werden zugemischt. Das Petriglas wird vorsichtig geschüttelt. Nach 2 Stunden werden erneut 0,025 mg des Natriumcyanoborhydrids zugegeben und vorsichtig solange geschüttelt, bis keine Bläschen mehr aufsteigen. Die Zugabe von Natriumborhydrid wird 4mal in gleicher Weise wiederholt. Danach wird das Reagenz über 72 Stunden stehen gelassen; schließlich in einem Überschuss PBS; pH=7,5 aufgenommen, dialysiert und gefriergetrocknet.

### Beispiel 4 (Verknüpfung einer aminierten HES mit Heparin und anschließende Umsetzung mit Humanalbumin)

200 g einer Hydroxyethylstärke (HES) [mittleres Molekulargewicht Mw = 50.000 ; DS = 0,3] wird zusammen mit einer 27%igen Ammoniumhydroxidlösung und 300 g eines Nickel/Kupfer/Chrom Katalysators mit einem Nickelanteil von 75%, einem Kupferanteil von 23% und einem Chromanteil von 2% in einen Autoclaven eingebracht. Unter Zugabe von Wasserstoff wird über einen Zeitraum von 12 Stunden mit Überdruck beaufschlagt. Die Temperatur wird bis auf 270 °C eingestellt. Anschließend wird das Gemisch entnommen, dialysiert und gefriergetrocknet. 200 mg Heparin¹⁾ werden in 5 ml PBS; pH= 7,5 gelöst und auf ein mit einem Bandgenerator positiv geladenes Petriglas pipettiert. 200 mg der reduktiv aminierten Hydroxyethylstärke werden in 10 ml destilliertes Wasser gelöst und vorsichtig zugegeben. Danach werden 0,025 mg Natriumcyanoborhydrid NaBH₃CN werden zugemischt. Das Petriglas wird vorsichtig geschüttelt. Nach 2 Stunden werden erneut 0,025 mg des Natriumcyanoborhydrids zugegeben und vorsichtig solange geschüttelt, bis keine Bläschen mehr aufsteigen. Die Zugabe von Natriumborhydrid wird 4 mal in gleicher Weise wiederholt.

Danach wird das Reagenz über 24 Stunden stehen gelassen. Nach erneuter Aufladung durch den Bandgenerator werden 10 mg Humanalbumin in 10 ml PBS (pH=7,5) zugegeben. Anschließend werden 0,025 mg Natriumcyanoborhydrid NaBH₃CN zugemischt. Die Petrischale wird vorsichtig geschüttelt. Die Zugabe von 0,025 mg Natriumcyanoborhydrid NaBH₃CN mit anschließendem Schütteln 4 mal mit und 4 mal ohne laufenden Bandgenerator wiederholt. Das Reagenz wird schließlich in einem Überschuss PBS (pH 7,5) aufgenommen, dialysiert und gefriergetrocknet.

### Beispiel 5 (Verknüpfung von Hydroxyethylstärke mit Heparin über Hexan-1,6-diamin)

### a) Tosylierung der Hydroxyethylstärke

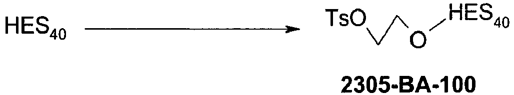

HES₄₀³⁾ (20 g) wird in Pyridin (200 ml) suspendiert und unter Rückfluss erhitzt bis eine klare Lösung entsteht. Danach kühlt man die Lösung auf 0°C ab und gibt unter Rühren Tosylchlorid (19.4 g, 200 Äqu.) in Portionen zu und lässt die Reaktionslösung langsam auf RT erwärmen. Man gibt die Reaktionslösung unter Rühren auf Acetonitril (500 ml). Es bildet sich sofort ein weisser Niederschlag, den man abfiltriert und im Vakuum trocknet. Man koevaporiert den erhaltenen weissen Schaum 3 x mit Acetonitril, nimmt den Rückstand in dest. Wasser auf und dialysiert 24h. Nach dem Eindampfen des Wassers wird die Titelverbindung als farbloser Feststoff erhalten (2.6 g). Das ¹H-NMR-Spektrum (400MHz, D₂O) zeigt gegenüber der reinen HES₄₀ zusätzlich typische symmetrische aromatische CH-Signale, bei einer chemischen Verschiebung zwischen 7-8ppm die auf Tosyl-Gruppen hinweisen.

### b) Substitution der tosylierten HES mit Amino-Linker

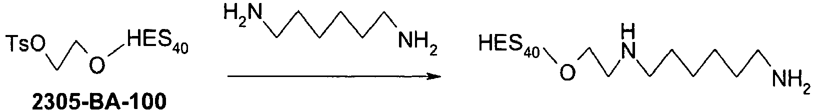

Eine Lösung aus 2305-BA-100 (3.3 g) und Hexandiamin (10.0 g, 1000 Äqu.) in DMF (5 ml) wird wird bei 50°C über Nacht gerührt und danach auf Aceton (300 ml) gegossen. Der ausgefallene Feststoff wird filtriert und getrocknet. Zur weiteren Reinigung wird das Rohprodukt in dest. Wasser gelöst und 24h dialysiert. Nach dem Eindampfen des Wassers wird das o.g. Reaktionsprodukt als farbloser Feststoff erhalten (1.5 g). Das ¹H-NMR-Spektrum (400MHz, D₂O) ergibt gegenüber Heparin zusätzlich typische CH₂-Signale, bei einer chemischen Verschiebung zwischen 1-2ppm die auf den Aminolinker hinweisen.

### c) EDC⁴⁾-Kopplung des in Schritt b) erhaltenen Reaktionsprodukts mit HEP¹⁾

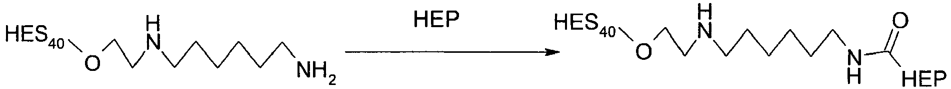

Zu einer Lösung aus HEP (60 mg) und dem in Schritt b) erhaltenen Produkt (200 mg) in destilliertem Wasser (4 ml) wird EDC⁴⁾ Hydrochlorid (80 mg, 100 Äqu.) gegeben. Die Reaktionslösung wird bei RT über Nacht gerührt und danach auf Aceton (5 ml) gegossen. Der ausgefallene Feststoff wird filtriert und getrocknet. Zur weiteren Reinigung wird das Rohprodukt in destilliertem Wasser gelöst und 24h dialysiert. Nach dem Eindampfen des Wassers wird das im Reaktionsschema dargestellte Verknüpfungsprodukt aus HES und Heparin als farbloser Feststoff erhalten (0.11 g).

### Beispiel 6 (Verknüpfung von Hydroxyethylstärke mit Heparin über Hexan-1,6-diamin)

### a) EDC⁴⁾-Kopplung von Heparin (HEP) mit Amino-Linker

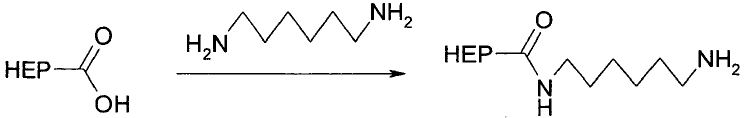

Zu einer Lösung aus HEP (1.0 g) und Hexan-1,6-diamin (0.8 g, 100 Äqu.) in destilliertem Wasser (10 ml) wird EDC Hydrochlorid⁴⁾ (14 g, 100 Äqu.) gegeben.

Die Reaktionslösung wird bei 20 °C über Nacht gerührt und danach auf Aceton (20 ml) gegossen. Der ausgefallene Feststoff wird filtriert und getrocknet. Es wird mittels LC-MS festgestellt, dass im Reaktionsprodukt noch nicht reagiertes Hexandiamin enthalten ist. Zur weiteren Reinigung wird das Rohprodukt in destilliertes Wasser gelöst und 24h dialysiert. Nach dem Eindampfen des Wassers wird das im Reaktionsschema dargestellte Kopplungsprodukt als farbloser Feststoff erhalten (0.8g). Das ¹H-NMR-Spektrum (400MHz, D₂O) ergibt gegenüber Heparin zusätzlich typische CH2-Signale bei einer chemischen Verschiebung zwischen 1-2ppm.

### b) Nukleophile Substitution des in Schritt a) erhaltenen Kopplungsprodukts mit der tosylierten HES aus Beispiel 5 Schritt a)

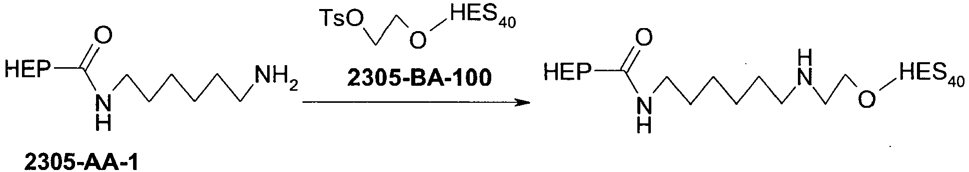

Zu einer Suspension aus 2305-AA-1 (30 mg) und 2305-BA100 (100 mg, MW: ca. 50 kDa) in DMSO (4 ml) wird Et₃N (0.003 ml, 100 Äqu.) zugespritzt und unter Rühren auf 80°C erhitzt. Das Reaktionsgemisch wird 6 h gerührt und danach auf Aceton (6 ml) gegossen. Der ausgefallene Feststoff wird filtriert und getrocknet. Man erhält die Titelverbindung als leicht beigen Feststoff (0.1 g). Das ¹H-NMR-Spektrum (400MHz, D₂O) ergibt gegenüber Heparin zusätzlich typische CH₂-Signale bei einer chemischen Verschiebung zwischen 1-2ppm.

### Beispiel 7 (Verknüpfung einer aminierten Hydroxyethylstärke mit Heparin über eine reduktive Aminierung)

### a) Aminierung der Hydroxyethylstärke (HES)

HES₄₀ → HES₄₀-NH₂

HES₄₀ (5.1 g, MW: 40 kDa) wird in einer wässrigen Ammoniumhydroxid-Lösung (100 ml, 22%ig) gelöst. Der Katalysator, bestehend aus Nickel (5.6 g, 325 mesh), Chrom (0.15 g, 100 mesh) und Kupfer (1.8 g, 1 micron) wird zur Lösung gegeben. Das Gemisch wird unter einer Wasserstoffatmosphäre bei 120°C im Autoklav 48 h gerührt. Nach dem Abkühlen auf 20 °C filtriert man den Katalysator ab und giesst das Filtrat auf Ethanol (20 ml). Der ausgefallene Niederschlag wird filtriert, mit wenig Ethanol/ Wasser gewaschen und getrocknet. Man erhält die aminierte HES als leicht bläulichen Feststoff (1.2 g).

### b) Reduktive Aminierung der in Schritt a) erhaltenen aminierten HES mit Heparin

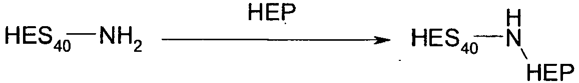

HEP (200 mg) wird in einer wässrigen Phosphatpuffer-Lösung (5 ml, pH=7.5) gelöst und eine Lösung der aminierten Hydroxyethylstärke aus Schritt a) (200 mg) in destilliertem Wasser (10 ml) zugetropft. Zur Reaktionslösung gibt man in Abständen von 2 h sechsmal NaCNBH₃ (jeweils 0.025 mg, aus einer wässrigen Stammlösung). Das Reaktionsgemisch wird nochmals 2 h bei 20 °C gerührt. Zur weiteren Reinigung wird das Rohprodukt 24h dialysiert. Nach dem Eindampfen des Wassers wird das im Reaktionsschema dargestellte Verknüpfungsprodukt aus Heparin und aminierter Hydroxyethylstärke als farbloser Feststoff erhalten (250 mg).

### Beispiel 8 (Verknüpfung eines fluoreszenzmarkierten Heparins mit einer aminierten Hydroxyethylstärke (HES) durch reduktive Aminierung

### a) Kopplung von Heparin (HEP) mit dem Fluoreszenzmarker 2-Aminopyridin

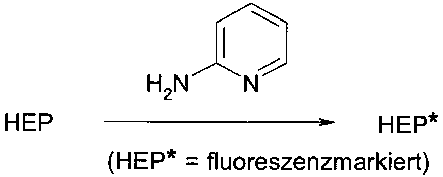

Zu einer Lösung aus 2-Amino-pyridin (31.7 g, 0.33 Mol, 1000 Äqu.) und NaCNBH₃ (2.1 g, 0.033 Mol, 100 Äqu.) in Formamid (50 ml) wird Heparin (5.0 g) gegeben. Die erhaltene Suspension rührt man bei 37°C über Nacht, wobei langsam eine klare Lösung entsteht. Die Reaktionslösung wird auf EtOH (50 ml) gegossen. Der ausgefallene Feststoff wird filtriert und getrocknet. Man erhält das im Reaktionschema aufgeführte Kopplungsprodukt (HEP*) als leicht beigen Feststoff (1.3 g). Das Kopplungsprodukt zeigt in wässriger Lösung als auch als Feststoff eine intensive blau-violette Fluoreszenz bei Bestrahlung mit UV-Licht mit 366 nm. Das ¹H-NMR-Spektrum (400MHz, D₂O) ergibt gegenüber Heparin zusätzlich typische aromatische CH-Signale bei einer chemischen Verschiebung zwischen 6.6-7.8ppm was auf typische Pyridin-Substituenten hinweist.

### b) Verknüpfung des in Schritt a) hergestellten fluoreszenzmarkierten Heparins (HEP*) mit der aus Beispiel 7 Schritt a) hergestellten aminierten Hydroxyethylstärke durch reduktive Aminierung

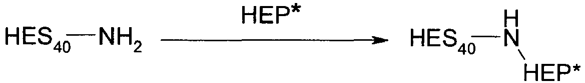

HEP* aus Schritt a)(200 mg, mittleres Molekulargewicht: 15 kDa) wird in einer wässrigen Phosphatpuffer-Lösung (5 ml, pH=7.5) gelöst und eine Lösung der aminierten HES aus Beispiel 7 Schritt a) (200 mg) in destilliertem Wasser (10 ml) zugetropft. Zur Reaktionslösung gibt man in Abständen von 2 h dreimal NaCNBH₃ (jeweils 0.025 mg, aus einer wässrigen Stammlösung). Das Reaktionsgemisch wird nochmals 2 h bei 20 °C gerührt. Zur weiteren Reinigung wird das Rohprodukt 24h dialysiert. Nach dem Eindampfen des Wassers wird das Verknüpfungsprodukt aus aminierter HES und fluoreszenzmarkiertem Heparin als farbloser Feststoff erhalten (200 mg).

Die Verbindung zeigt in wässriger Lösung als auch als Feststoff eine intensive grün-gelbe Fluoreszenz bei Bestrahlung mit UV-Licht mit 366nm. Das ¹H-NMR-Spektrum (400MHz, D₂O) ergibt gegenüber Heparin zusätzlich typische aromatische CH-Signale bei einer chemischen Verschiebung zwischen 7.0-7.8 ppm was auf typische Pyridin-Substituenten hinweist.

### Beispiel 9 Verknüpfung des in Beispiel 5 Schritt b) erhaltenen Produkts mit dem fluoreszenzmarkierten Heparin aus Beispiel 8 Schritt a) durch Kopplung mittels EDC⁴⁾

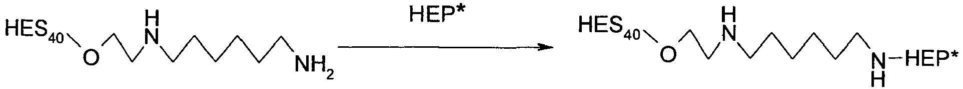

Zu einer Lösung aus HEP* (Beispiel 8, Schritt a))(60 mg) und dem Reaktionsprodukt aus Beispiel 5, Schritt b) (160 mg) in destilliertem Wasser (4 ml) wird EDC Hydrochlorid (80 mg, 100 Äqu.) gegeben. Die Reaktionslösung wird bei 20 °C über Nacht gerührt und danach auf Aceton (5 ml) gegossen. Der ausgefallene Feststoff wird filtriert und getrocknet. Zur weiteren Reinigung wird das Rohprodukt in destilliertem Wasser gelöst und 24h dialysiert. Nach dem Eindampfen des Wassers wird das gewünschte Verknüpfungprodukt gemäß dem aufgeführten Formelschema als farbloser Feststoff erhalten (0.1g).

Die Verbindung zeigt in wässriger Lösung als auch als Feststoff eine intensive grün-gelbe Fluoreszenz bei Bestrahlung mit UV-Licht mit 366 nm. Das ¹H-NMR-Spektrum (400MHz, D₂O) ergibt gegenüber Heparin zusätzlich typische aromatische CH-Signale bei einer chemischen Verschiebung zwischen 7.0-7.8ppm was auf typische Pyridin-Substituenten hinweist.
1) Heparin (auch als HEP abgekürzt): eingesetzt wurde das Natrium-Salz (vom Schwein, porcine origin), pH = 7, mittleres M_{w} = 12-15 kDa, Hersteller: Changzhou Qianhong Bio-Pharma Co., Ltd., Jiangsu, China.
2) A. N. De Belder und Kirsti Granath; Carbohydrate Research, 30 (1973), 375-378.
3)HES₄₀: Hydroxyethylstärke mit mittlerem Molekulargewicht M_{w} = 40 kDa, Substitutionsgrad DS = 0,3; Hersteller: Fa. BBraun, Crissier, Schweiz.
4) EDC: N-Dimethylaminopropyl-N-ethylcarbodiimid-Hydrochlorid

## Patentansprüche

1. Verbindung der allgemeinen Formel (I)
(T-Z)ₙ-P (I),
wobei
• T ein Glykosaminoglykan als Transportvermittler,
• P eine kolloidwirksame Verbindung, ausgewählt aus Hydroxyalkylstärke und Carboxyalkylstärke,
• Z ein erster Linker, durch den T und P kovalent verknüpft ist, und
• n eine ganze Zahl, die mindestens 1 ist,
und wobei der Transportvermittler T und/oder das Kolloid P
m Gruppen -(L-A) trägt, wobei
• A ein Arzneimittelwirkstoff oder ein Fluoreszenzmarker,
• L ein zweiter Linker, durch den P mit A kovalent verknüpft ist, oder durch den T mit A kovalent verknüpft ist und
• m eine ganze Zahl, die mindestens 1 ist,
• und wobei der Arzneimittelwirkstoff A ausgewählt ist aus der Gruppe bestehend aus Antibiotika, Chemotherapeutika, Cytostatika und cytotoxischen Substanzen.

2. Verbindung gemäß Anspruch 1, **dadurch gekennzeichnet, dass** der Transportvermittler T ein Glykosaminoglykan ist, welches ausgewählt ist aus der Gruppe bestehend aus Heparin und Heparinsulfat.

3. Verbindung gemäß Anspruch 1 oder 2, **dadurch gekennzeichnet, dass** das Glykosaminoglykan Heparin oder Heparinsulfat mit weniger als 6 Saccharideinheiten ist.

4. Verbindung gemäß mindestens einem der Ansprüche 1 bis 3, **dadurch gekennzeichnet, dass** die kolloidwirksame Verbindung ausgewählt ist aus der Gruppe bestehend aus Hydroxyalkylstärken, Carboxyalkylstärken, Hydroxyalkylcarboxyalkylstärke, aminierte Hydroxyalkylstärke, aminierte Hydroxyalkylcarboxyalkylstärke und aminierte Carboxyalkylstärke.

5. Verbindung gemäß Anspruch 4, **dadurch gekennzeichnet, dass** die kolloidwirksame Verbindung ausgewählt ist aus Hydroxyethylstärke oder aminierter Hydroxyethylstärke.

6. Verbindung gemäß Anspruch 4 oder 5, **dadurch gekennzeichnet, dass** die kolloidwirksame Verbindung ein mittleres Molekulargewicht von 20000 bis 800000 Dalton, vorzugsweise 25000 bis 500000 Dalton, insbesondere von 30000 bis 200000 aufweist.

7. Verbindung gemäß mindestens einem der Ansprüche 4 bis 6, **dadurch gekennzeichnet, dass** der Substitutionsgrad DS der Hydroxyethylstärke, zwischen 0,2 und 0,8, vorzugsweise zwischen 0,3 und 0,6 liegt.

8. Verbindung gemäß mindestens einem der Ansprüche 1 bis 7, **dadurch gekennzeichnet, dass** der Fluoreszenzmarker ausgewählt ist aus der Gruppe bestehend aus Fluoresceinisothiocyanat (FITC), Phycoerythrin, Rhodamid und 2-Aminopyridin.

9. Verbindung gemäß mindestens einem der Ansprüche 1 bis 8, erhältlich durch reduktive Aminierung eines freie Aminogruppen (-NH₂) aufweisenden Kolloids P, ausgewählt aus der Gruppe bestehend aus aminierter Stärke, aminierter Hydroxyalkylstärke, aminierter Hydroxyalkylcarboxyalkylstärke und aminierter Carboxyalkylstärke, mit dem Glykosaminoglykan als Transportvermittler T, der mindestens eine Aldehyd- oder Ketogruppe aufweist und wobei das Kolloid P und/oder der Transportvermittler T mit m Resten -(L-A) verknüpft ist.

10. Verbindung gemäß Anspruch 9, **dadurch gekennzeichnet, dass** der Transportvermittler Heparin oder ein Heparinderivat ist.

11. Verbindung gemäß Anspruch 1, **dadurch gekennzeichnet, dass** der Transportvermittler T Heparin und das Kolloid P eine Hydroxyethylstärke ist und der erste Linker Z eine -NH-Gruppe darstellt.

12. Pharmazeutische Zubereitung, umfassend die Verbindung gemäß mindestens einem der Ansprüche 1 bis 11.

13. Pharmazeutische Zubereitung gemäß Anspruch 12, **dadurch gekennzeichnet, dass** die Zubereitung wässrig ist und injizierbar.

14. Verfahren zur Herstellung einer Verbindung der allgemeinen Formel (I) gemäß mindestens einem der Ansprüche 1-11, durch Verknüpfen eines Glykosaminoglykans als Transportvermittlers T mit einer kolloidwirksamen Verbindung P, ausgewählt aus Hydroxyalkylstärke und Carboxyalkylstärke, unter Ausbildung eines Linkers Z, durch den T und P kovalent miteinander verknüpft sind und wobei das Kolloid P und/oder der Transportvermittler T mit m Resten - (L-A) verknüpft ist.

15. Verfahren gemäß Anspruch 14, **dadurch gekennzeichnet, dass** in einem ersten Schritt das Kolloid P ausgewählt ist aus der Gruppe bestehend aus aminierter Stärke, aminierter Hydroxyalkylstärke, aminierter Hydroxyalkylcarboxyalkylstärke und aminierter Carboxyalkylstärke mit einem Transportvermittler T, ausgewählt aus der Gruppe der Heparine oder Heparinderivate in Gegenwart eines Reduktionsmittels, ausgewählt aus der Gruppe bestehend aus LiAlH₄, LiBH₄, NaBH₄ und NaBH₃CN, zur Reaktion gebracht wird.

## Claims

1. A compound of general formula (I)
(T-Z)ₙ-P (I),
wherein
• T is a glycosaminoglycan transport mediator;
• P is a colloid-active compound selected from hydroxyalkyl starch and carboxyalkyl starch;
• Z is a first linker by means of which T and P are covalently linked together; and
• n is an integer of at least 1;
and wherein the transport mediator T and/or the colloid P bears m groups -(L-A), wherein
• A is a medicinally active substance or a fluorescence marker;
• L is a second linker through which P is covalently linked with A, or through which T is covalently linked with A; and
• m is an integer of at least 1; and
• wherein said medicinally active compound A is selected from the group consisting of antibiotics, chemotherapeutics, cytostatic agents and cytotoxic substances.

2. The compound according to claim 1, **characterized in that** said transport mediator T is a glycosaminoglycan selected from the group consisting of heparin and heparin sulfate.

3. The compound according to claim 1 or 2, **characterized in that** said glycosaminoglycan is heparin or heparin sulfate with less than 6 saccaride units.

4. The compound according to at least one of claims 1 to 3, **characterized in that** said colloid-active compound is selected from the group consisting of hydroxyalkyl starches, carboxyalkyl starches, hydroxyalkyl carboxyalkyl starch, aminated hydroxyalkyl starch, aminated hydroxyalkyl carboxyalkyl starch and aminated carboxyalkyl starch.

5. The compound according to claim 4, **characterized in that** said colloid-active compound is selected from hydroxyethyl starch or aminated hydroxyethyl starch.

6. The compound according to claim 4 or 5, **characterized in that** said colloid-active compound has an average molecular weight of from 20,000 to 800,000 daltons, preferably from 25,000 to 500,000 daltons, especially from 30,000 to 200,000 daltons.

7. The compound according to at least one of claims 4 to 6, **characterized in that** the degree of substitution, DS, of the hydroxyethyl starch is from 0.2 to 0.8, preferably from 0.3 to 0.6.

8. The compound according to at least one of claims 1 to 7, **characterized in that** said fluorescence marker is selected from the group consisting of fluorescein isothiocyanate (FITC), phycoerythrin, rhodamide and 2-amino-pyridine.

9. The compound according to at least one of claims 1 to 8, obtainable by reductive amination of a colloid P having free amino groups (-NH₂) selected from the group consisting of aminated starch, aminated hydroxyalkyl starch, aminated hydroxyalkyl carboxyalkyl starch, and aminated carboxyalkyl starch with said glycosaminoglycan transport mediator T having at least one aldehyde or keto group, and wherein the colloid P and/or transport mediator T is linked with m units -(L-A).

10. The compound according to claim 9, **characterized in that** said transport mediator is heparin or a heparin derivative.

11. The compound according to claim 1, **characterized in that** said transport mediator T is heparin, and said colloid P is a hydroxyethyl starch, and the first linker Z is an -NH- group.

12. A pharmaceutical formulation comprising the compound according to at least one of claims 1 to 11.

13. The pharmaceutical formulation according to claim 12, **characterized in that** said formulation is aqueous and injectable.

14. A process for preparing a compound of general formula (I) according to at least one of claims 1 to 11 by linking a glycosaminoglycan transport mediator T with a colloid-active compound P selected from hydroxyalkyl starch and carboxyalkyl starch to form a linker Z through which T and P are covalently linked with one another, and wherein the colloid P and/or the transport mediator T is linked with m units -(L-A).

15. The process according to claim 14, **characterized in that**, in a first step, the colloid P, which is selected from the group consisting of aminated starch, aminated hydroxyalkyl starch, aminated hydroxyalkyl carboxyalkyl starch, and aminated carboxyalkyl starch, is reacted with a transport mediator T selected from the group of heparins or heparin derivatives in the presence of a reducing agent selected from the group consisting of LiAlH₄, LiBH₄, NaBH₄ and NaBH₃CN.

## Revendications

1. Composé répondant à la formule générale (I)
(T-Z)ₙ-P (I),
où
• T est un glycosaminoglycan comme médiateur de transport,
• P est un composé à activité colloïde choisi parmi amidon hydroxyalkylé et amidon carboxyalkylé,
• Z est un premier linker, par lequel T et P sont liés de manière covalente, et
• n est un nombre entier d'au moins 1,
et dans lequel ledit médiateur de transport T et/ou le colloïde P porte m groupes -(L-A), où
• A est un principe actif médicinal ou un marqueur fluorescent,
• L est un second linker, par lequel P est lié avec A de manière covalente, ou par lequel T est lié avec A de manière covalente, et
• m est un nombre entier d'au moins 1, et
• dans lequel le principe actif médicinal A est choisi dans le groupe consistant en antibiotiques, agents chimiothérapeutiques, agents cytostatiques et substances cytotoxiques.

2. Composé selon la revendication 1, **caractérisé en ce que** ledit médiateur de transport T est un glycosaminoglycan choisi dans le groupe consistant en héparine et sulfate d'héparine.

3. Composé selon la revendication 1 ou 2, **caractérisé en ce que** ledit glycosaminoglycan est une héparine ou un sulfate d'héparine ayant moins de 6 unités saccharides.

4. Composé selon l'une au moins des revendications 1 à 3, **caractérisé en ce que** ledit composé à activité colloïde est choisi dans le groupe consistant en amidons hydroxyalkylés, amidons carboxyalkylés, amidons hydroxyalkylés-carboxyalkylés, amidons hydroxyalkylés aminés, amidons hydroxyalkylés-carboxyalkylés aminés, et amidons carboxyalkylés aminés.

5. Composé selon la revendication 4, **caractérisé en ce que** ledit composé à activité colloïde est choisi parmi l'amidon hydroxyéthylé ou l'amidon hydroxyéthylé aminé.

6. Composé selon la revendication 4 ou 5, **caractérisé en ce que** ledit composé à activité colloïde présente un poids moléculaire moyen de 20 000 à 800 000 daltons, de préférence 25 000 à 500 000 daltons, notamment 30 000 à 200 000 daltons.

7. Composé selon l'une au moins des revendications 4 à 6, **caractérisé en ce que** le degré de substitution DS de l'amidon hydroxyéthylé est entre 0,2 et 0,8, de préférence entre 0,3 et 0,6.

8. Composé selon l'une au moins des revendications 1 à 7, **caractérisé en ce que** ledit marqueur fluorescent est choisi dans le groupe consistant en isothiocyanate de fluorescéine (FITC), phycoérythrine, rhodamide et 2-aminopyridine.

9. Composé selon l'une au moins des revendications 1 à 8, disponible par l'amination réductrice d'un colloïde P présentant des groupes aminés libres (-NH₂), choisi dans le groupe consistant en amidon aminé, amidon hydroxyalkylé aminé, amidon hydroxyalkylé-carboxyalkylé aminé, et amidon carboxyalkylé aminé, avec ledit glycosaminoglycan comme médiateur de transport T présentant au moins un groupe aldéhyde ou cétone, et dans lequel ledit colloïde P et/ou ledit médiateur de transport T est lié à m résidus -(L-A).

10. Composé selon la revendication 9, **caractérisé en ce que** ledit médiateur de transport est l'héparine ou un dérivé de l'héparine.

11. Composé selon la revendication 1, **caractérisé en ce que** le médiateur de transport T est l'héparine, et le colloïde P est un amidon hydroxyéthylé, et le premier linker Z représente un groupe -NH-.

12. Formulation pharmaceutique comprenant le composé selon l'une au moins des revendications 1 à 11.

13. Formulation pharmaceutique selon la revendication 12, **caractérisée en ce que** la formulation est aqueuse et injectable.

14. Procédé pour préparer un composé de la formule générale (I) selon l'une au moins des revendications 1 à 11 par la liaison d'un glycosaminoglycan comme médiateur de transport T avec un composé à activité colloïde P choisi parmi amidon hydroxyalkylé et amidon carboxyalkylé pour former un linker Z, par lequel T et P sont liés de manière covalente, et dans lequel le colloïde P et/ou le médiateur de transport T est lié à m résidus -(L-A).

15. Procédé selon la revendication 14, **caractérisé en ce que**, dans une première étape, l'on fait réagir le colloïde P, choisi dans le groupe consistant en amidon aminé, amidon hydroxyalkylé aminé, amidon hydroxyalkylé-carboxyalkylé aminé, et amidon carboxyalkylé aminé, avec un médiateur de transport T choisi dans le groupe des héparines ou des dérivés de l'héparine en présence d'un agent réducteur choisi dans le groupe consistant en LiAlH₄, LiBH₄, NaBH₄ et NaBH₃CN.
